# EUROPÄISCHE PATENTSCHRIFT

(11) **EP 3 128 944 B1**
(45) Veröffentlichungstag und Bekanntmachung des Hinweises auf die Patenterteilung: **22.05.2019**
(21) Anmeldenummer: 15715253.9
(22) Anmeldetag: 09.04.2015
(51) Int. Cl.: A61C 13/00, A61C 13/10, A61C 13/12, A61C 13/08

(54) **VERFAHREN ZUR BEARBEITUNG VON VORKONFEKTIONIERTEN PROTHESENZÄHNEN**
METHOD FOR PROCESSING PREFABRICATED PROSTHETIC TEETH
PROCÉDÉ DE FAÇONNAGE DE DENTS PROTHÉTIQUES PRÉCONFECTIONNÉES

(30) Priorität: 11.04.2014 DE 102014105190
(43) Veröffentlichungstag der Anmeldung: 15.02.2017
(73) Patentinhaber: Kulzer GmbH, 63450 Hanau (DE)
(72) Erfinder: SAVIC, Novica, 63526 Erlensee (DE); RENZ, Karl-Heinz, 63755 Alzenau (DE); GALL, Silke Maren, 63755 Alzenau (DE)
(74) Vertreter: Schultheiss & Sterzel Patentanwälte PartG mbB
(86) Internationale Anmeldenummer: PCT/EP2015/057704
(87) Internationale Veröffentlichungsnummer: WO 2015/155283

(56) Entgegenhaltungen:
- EP-A1- 2 742 907
- WO-A1-2013/124452
- JP-A- 2005 253 756
- US-A1- 2012 276 502

## Beschreibung

Die Erfindung betrifft ein Verfahren zur Bearbeitung von Prothesenzähnen zur Herstellung einer Totalprothese oder wenigstens einer Teilprothese mit zumindest zwei Prothesenzähnen.

Die Erfindung betrifft auch eine Vorrichtung zur Halterung von Prothesenzähnen zur Durchführung eines solchen Verfahrens und ein Set zur Durchführung eines solchen Verfahrens mit einer solchen Vorrichtung.

Die Erfindung betrifft somit die Herstellung von konfektionierten Prothesenzähnen zur Weiterverarbeitung für partielle Kunststoffprothesen (Teilprothesen) und totale Kunststoffprothesen (Totalprothesen), die maschinell im CAM-Verfahren (CAM - Computer-Aided Manufacturing, Deutsch: rechner-unterstützte Fertigung) produziert werden. Bevorzugt werden die Prothesen computergestützt mit CAD-Verfahren (CAD - Computer-Aided Design, Deutsch: rechnerunterstützte Konstruktion) konstruiert. Die Prothesenzähne können als Halbzeug zur teilweisen oder vollständigen Herstellung von Kunststoffprothesen mit CAM-Verfahren bereitgestellt werden.

Der derzeit gängige Weg ist die analoge Erstellung von Prothesenzähnen. Dabei werden die Prothesenzähne manuell und einzeln auf einer Wachsbasis aufgestellt. Diese Wachsprothese wird im nächsten Schritt in einer Küvette mit Gips eingebettet, um dann nach Aushärten des Gipses die Wachsbasis mit heißem Wasser herauszuwaschen und einen Hohlraum für den Prothesenkunststoff zu schaffen. Die Prothesenzähne verbleiben dabei im Gips. Ein entsprechender Gips wird in den Hohlraum injiziert oder "gestopft" und man erhält nach Aushärtung des Kunststoffs die Prothese beziehungsweise den fertigen Prothesenzahn.

Aus der DE 10 2005 013 459 B4 ist eine Aufstellhilfe zur Bearbeitung von Zahnblöcken bekannt, bei dem die Ausrichtung von Prothesenzähnen zur Optimierung der Okklusion der Prothesenzähne eingestellt werden kann. Bei der Aufstellung vorkonfektionierter Prothesenzähne werden diese der jeweiligen Mundsituation des Patienten vom Zahntechniker angepasst und beschliffen. Es gibt bereits erste Verfahren, wie beispielsweise die aus der DE 10 2009 056 752 A1 oder der WO 2013 124 452 A1 bekannten Verfahren, bei denen die Teil- beziehungsweise Totalprothese digital aufgestellt und über CAD-CAM-Verfahren produziert wird.

Nachteilig ist hieran, dass die korrekte Positionierung der Prothesenzähne relativ zueinander in einer Prothesenbasis schwierig ist und dass es dabei zu Fehlern kommen kann. In der Regel wird eine passgenaue Mulde in einer Prothesenbasis vorgefertigt, um dann die konfektionierten Prothesenzähne einzukleben. Das funktioniert jedoch nur, wenn ausreichend Platz vorhanden ist und die vorkonfektionierten Prothesenzähne nicht von unten (basal) angeschliffen werden müssen. Das ist eher die Ausnahme. In den meisten Fällen muss jeder Prothesenzahn aus Platzgründen beschliffen werden. Vorkonfektionierte Prothesenzähne sind im Rahmen der Erstellung von prothetischen Arbeiten in der Regel mindestens von basal zu bearbeiten. Bei manueller Umsetzung erfolgt die Bearbeitung einzeln pro Prothesenzahn durch den Verarbeiter. In diesem Fall passt er aber nicht mehr in die vorproduzierte Mulde in der Prothesenbasis.

Die DE 10 2011 101 678 A1 schlägt vor, dass der Kronenbereich eines Prothesenzahns in eine Trägerschicht eingebettet wird, um den Prothesenzahn anschließend zu bearbeiten. Dadurch soll eine feste und präzise Verbindung bei gleichzeitig definierter Positionierung der Prothesenzähne erzielt werden. Nachteilig ist hieran, dass der Prothesenzahn bereichsweise freigelegt werden muss, um den eingebetteten Kronenbereich zu bearbeiten. Die Nachteile des Verfahrens liegen auch in der aufwendigen Herstellung der Körper. Diese sind einzeln oder in Gruppen durch Einsetzen der fertigen Prothesenzähne in einen Halter, Aufsetzen eines Außenrahmens und abschließendes Ausgießen mit einem Verbindungsmedium (z.B. Wachs) herzustellen. Dadurch ist das Eingießen der Prothesenzähne mit der die Trägerschicht bildenden Masse handwerklich aufwendig.

Die Trägerschicht muss aushärten, bevor der Prothesenzahn bearbeitet werden kann, da insbesondere bei der Verwendung von automatisierten CAM-Fräsmaschinen erhebliche Kräfte auftreten. Die Positionsgenauigkeit des Verfahrens leidet zudem unter möglichen Verformungen der noch nicht vollständig ausgehärteten Trägerschicht, so dass im Extremfall der nachbearbeitete Prothesenzahn nicht exakt genug ausgefräst wird und deswegen entsorgt werden muss. Ferner kann eine solche Trägerschicht meist nur mit einem gewissen Aufwand wieder vollständig von dem Prothesenzahn entfernt werden. Das Material zur Herstellung der Trägerschicht muss vorgehalten und eventuell vor der Anwendung gemischt, geschmolzen beziehungsweise aktiviert werden. Nach der Verwendung muss es entsorgt werden. Ferner kann es bei dem Einsetzen der einzelnen bearbeiteten Prothesenzähne zu Abweichungen der Position und der Ausrichtung der Prothesenzähne zueinander kommen.

Die Aufgabe der Erfindung besteht also darin, die Nachteile des Stands der Technik zu überwinden. Insbesondere soll ein Verfahren und eine Vorrichtung bereitgestellt werden, mit dem eine möglichst einfache, vollständige und kostengünstige Bearbeitung der Prothesenzähne beziehungsweise Herstellung der Prothese möglich ist. Dabei sollen möglichst wenige Nachbearbeitungsschritte notwendig sein. Möglichst viele notwendige Teile sollen wiederverwendbar sein. Eine Nachbearbeitung der fertig nachbearbeiteten Prothesenzähne soll vermieden werden. Zudem sollen Fehlermöglichkeiten beim Einsetzen der Prothesenzähne in die Prothesenbasis vermieden werden. Das gesamte Verfahren soll auch einfach und so weit wie möglich maschinell und automatisch durchgeführt werden können.

Die Aufgaben der Erfindung werden gelöst durch ein Verfahren zur Bearbeitung von Prothesenzähnen zur Herstellung einer Totalprothese oder wenigstens einer Teilprothese mit zumindest zwei Prothesenzähnen aufweisend die folgenden Verfahrensschritte:
1) Wenigstens zwei vorkonfektionierte Prothesenzähne werden in eine einteilige elastische Form eingesetzt, wobei die koronalen Seiten der Prothesenzähne in Ausnehmungen der elastischen Form eingesteckt werden,
2) eine Klemmvorrichtung wird an der elastischen Form angebracht oder ist an der elastischen Form angebracht und mit der Klemmvorrichtung wird nach dem Einsetzen der Prothesenzähne in die elastische Form ein mechanischer Druck auf die elastische Form ausgeübt, wobei der Druck über die elastische Form eine Kraft auf die in die elastische Form eingesetzten Prothesenzähne ausübt und dadurch die Prothesenzähne in der elastischen Form fixiert,
3) die Prothesenzähne werden in der elastischen Form derart zueinander positioniert und ausgerichtet, dass, spätestens nach Anbringen der Klemmvorrichtung an der elastischen Form, die Prothesenzähne relativ zueinander die gewünschte Position und die gewünschte Ausrichtung aufweisen, die sie in der herzustellenden Teilprothese oder in der herzustellenden Totalprothese haben sollen,
4) die elastische Form wird mit der Klemmvorrichtung und den Prothesenzähnen in einer definierten Position in einer Halterung einer CAM-Vorrichtung zum Abtragen von Material der Prothesenzähne mit einem CAM-Verfahren befestigt, und
5) wenigstens einer der in der elastischen Form fixierten Prothesenzähne wird mit einem CAM-Verfahren basal abgetragen.

Als Klemmvorrichtung im Sinne der vorliegenden Erfindung werden alle Vorrichtungen verstanden, mit denen ein mechanischer Druck auf die elastische Form ausgeübt werden kann. Bevorzugt wird die Kraft von mindestens zwei gegenüberliegenden Richtungen ausgeübt. Ebenfalls bevorzugt liegen die Richtungen der Kraftvektoren, die den Druck auf die elastische Form ausüben, in einer Ebene. Die Klemmvorrichtung kann bevorzugt aus einem Kunststoff bestehen. Zudem kann bevorzugt vorgesehen sein, dass die Elastizität des Materials der Klemmvorrichtung zum Ausüben des mechanischen Drucks verwendet wird.

Bevorzugt werden alle in der elastischen Form fixierten Prothesenzähne mit einem CAM-Verfahren basal abgetragen.

Bevorzugt wird wenigstens eine basale Seite zumindest eines in der elastischen Form fixierten Prothesenzahns computergesteuert mit dem CAM-Verfahren bearbeitet, insbesondere mit einer CAM-Vorrichtung bearbeitet.

Als elastische Form kann bevorzugt eine Kunststoffform verwendet werden. Besonders bevorzugt kann eine feste Silikonform verwendet werden. Die Silikonform muss elastisch sein, damit die Prothesenzähne eingesetzt werden können, aber gleichzeitig sehr hart sein, damit bei der Bearbeitung und den dabei auftretenden mechanischen Belastungen der Prothesenzähne deren Position und Ausrichtung unverändert bleibt.

Gemäß einer besonders bevorzugten Ausführungsform der vorliegenden Erfindung weist die elastische Form sechs Ausnehmungen auf, so dass sechs Prothesenzähne in einer elastischen Form bearbeitet werden können. Bevorzugt gehören alle Prothesenzähne zu einem Quadranten des Patienten.

Die elastische Form ist zur Umsetzung erfindungsgemäßer Verfahren einteilig aufgebaut. Zumindest im Bereich der Ausnehmungen zur Aufnahme der Prothesenzähne.

Es ist erfindungsgemäße bevorzugt, wenn mehrere Prothesenzähne in jeweils zwei separaten elastischen Formen mit zwei separaten Klemmvorrichtungen fixiert werden. Dies hat den Vorteil, dass dann anschließend mit einem einzigen Arbeitsschritt mehrere in den elastischen Formen gehaltene Prothesenzähne in einem Vorgang bearbeitet und auf ihre Okklusion geprüft werden können.

Ferner kann erfindungsgemäß vorgesehen sein, dass die in der elastischen Form fixierten Prothesenzähne, nachdem zumindest einer der Prothesenzähne basal abgetragen wurde, auf ihrer basalen Seite mit einer Prothesenbasis verbunden werden, wobei bevorzugt die Prothesenzähne mit ihrer basalen Seite in dafür vorgesehene Ausnehmungen der Prothesenbasis geklebt werden.

Hierdurch wird die Herstellung der Teilprothese oder der Teilprothesen oder der Totalprothese abgeschlossen. Die Ausnehmungen der Prothesenbasis sind bevorzugt als Vertiefungen in der Prothesenbasis angeordnet. Besonders bevorzugt passen die Seitenwände der Ausnehmungen zu der Außenfläche der vorkonfektionierten Prothesenzähne. Die Außenfläche der Prothesenzähne ist die Fläche, die die Zahnachse als Mantel umgibt, also die Fläche, die senkrecht zum koronalen und basalen Ende des Zahns beziehungsweise parallel zur Zahnachse angeordnet sind. Die Außenfläche ist bevorzugt die Zahnmantelfläche. Es reicht aus, wenn die vorkonfektionierten Prothesenzähne auf der basalen Seite eine zu den Seitenwänden der Vertiefung in der Prothesenbasis passende Außenform beziehungsweise Zahnmantelfläche aufweisen. Besonders bevorzugt weisen die vorkonfektionierten Prothesenzähne auf der basalen Seite eine zylindrische Geometrie auf. Die zylindrische Geometrie bezieht sich dabei auch auf allgemeine Zylinder, also nicht nur Zylinder mit kreisförmiger Grundfläche.

Dabei kann erfindungsgemäß vorgesehen sein, dass nach dem Verbinden der Prothesenzähne mit der Prothesenbasis die elastische Form von den Prothesenzähnen gelöst wird, bevorzugt nach dem Verbinden der Prothesenzähne mit der Prothesenbasis die Klemmvorrichtung gelöst wird oder von der elastischen Form gelöst wird und daran anschließend die elastische Form von den mit der Prothesenbasis verbundenen Prothesenzähnen gelöst wird.

Hierdurch wird erreicht, dass die Prothesenzähne in der gewünschten relativen Position und Ausrichtung zueinander in die Prothesenbasis eingesetzt werden.

Mit der Erfindung wird auch vorgeschlagen, dass die Prothesenzähne beim Einsetzen in die elastische Form darin derart zueinander positioniert und ausgerichtet werden, dass die Prothesenzähne zueinander die gewünschte Position und die gewünschte Ausrichtung aufweisen, die sie in der herzustellenden Teilprothese oder Totalprothese haben sollen.

Hierdurch erhalten die Prothesenzähne bereits beim Einsetzen in die elastische Form die gewünschte relative Position und Ausrichtung zueinander. Dies ist insbesondere deshalb sinnvoll, da die elastische Form möglichst steif sein soll, um eine Veränderung der Position und der Ausrichtung der Prothesenzähne in der elastischen Form beim Abtragen von Material an der Prothesenbasis zu vermeiden.

Es wird ferner vorgeschlagen, dass beim basalen Abtragen des zumindest einen Prothesenzahns die durch das Abtragen erzeugte basale Oberfläche des zumindest einen Prothesenzahns mit einem CAD-Verfahren berechnet wird.

Hierdurch wird eine weitere Automatisierung des Verfahrens zur Herstellung der Prothese beziehungsweise zur Positionierung und Bearbeitung der Prothesenzähne erreicht.

Des Weiteren kann dabei vorgesehen sein, dass bei der Berechnung der zu erzeugenden basalen Oberfläche des zumindest einen Prothesenzahns, bevorzugt aller in der elastischen Form fixierten Prothesenzähne, die Oberfläche der Prothesenbasis, mit der die Prothesenzähne verbunden werden sollen, und die bekannte Position und Ausrichtung des zumindest einen Prothesenzahns relativ zu dem anderen Prothesenzahn oder den anderen Prothesenzähnen rechnerisch berücksichtigt wird.

Mit einem solchen Verfahren kann eine weitere Automatisierung des gesamten Prozesses erreicht werden. Zudem wird so die Genauigkeit, mit der die Prothese hergestellt wird und damit die Qualität der erzeugten Teil- oder Totalprothese verbessert.

Dabei kann erfindungsgemäß wiederum vorgesehen sein, dass die Ausnehmung der Prothesenbasis, mit der die Prothesenzähne verbunden werden sollen, durch einen 3-Dimensionalen-Scan der Mundraum-Situation eines Patienten oder eines 3-Dimensionalen-Scans eines Abdrucks der Mundraum-Situation des Patienten und durch eine rechnergestützte virtuelle Aufstellung der Prothesenzähne in der Prothesenbasis bestimmt wird.

Auch mit diesem Verfahrensschritt wird eine weitere Automatisierung des Gesamtprozesses verbessert.

Mit einer Weiterentwicklung der Erfindung wird auch vorgeschlagen, dass die gewünschte Okklusion der Prothesenzähne zueinander oder der Prothesenzähne und der echten Zähne des Patienten zueinander rechnergestützt bestimmt wird und bei einer Berechnung der basalen Oberfläche des CAD-Modells des zumindest einen Prothesenzahns berücksichtigt wird. Es soll also die Okklusion bei der Berechnung der basalen Oberfläche des CAD-Modells des zumindest einen zu bearbeitenden Prothesenzahns berücksichtigt werden, bevorzugt natürlich bei der Bearbeitung aller Prothesenzähne.

Hierdurch kann eine Nachbearbeitung der erzeugten Teil- oder Totalprothese vermieden oder zumindest reduziert werden. Zusammen mit der gemeinsamen Aufstellung der Prothesenzähne in der elastischen Form oder den elastischen Formen, wenn mehrere Vorrichtungen zur Herstellung mehrerer Teilprothesen oder zur Herstellung einer Totalprothese angewendet werden, kann hierdurch eine hohe Genauigkeit der erzeugten Teilprothese(n) oder Totalprothese erreicht werden.

Bevorzugte Ausgestaltungen des Verfahrens können auch vorsehen, dass die Ausnehmungen in der elastischen Form derart tief sind, dass sie die eingesetzten Prothesenzähne an jeweils einem mittleren Umfang zumindest bereichsweise umschließen.

Hierdurch wird eine sichere Fixierung der Position und der Ausrichtung der Prothesenzähne zueinander und zur CAM-Vorrichtung ermöglicht. Dies ist insbesondere bei der basalen Bearbeitung der Prothesenzähne wichtig und von Vorteil.

Bevorzugte Verfahren können auch vorsehen, dass die Innenflächen der Ausnehmungen zumindest bereichsweise formschlüssig und flächenbündig zu Bereichen der Außenflächen der Prothesenzähne passen. Dabei kann bevorzugt vorgesehen sein, dass die Innenflächen der Ausnehmungen Negativen der Außenflächen der koronalen Seiten der Prothesenzähne entsprechen.

Hierdurch kann eine besonders stabile Fixierung der Prothesenzähne in der elastischen Form erreicht werden, insbesondere auch bei einer mechanischen Bearbeitung der basalen Seiten der Prothesenzähne.

Mit einer Weiterentwicklung der Erfindung wird vorgeschlagen, dass die elastische Form mit einer Halteeinrichtung der Klemmvorrichtung in der definierten Position in der Halterung einer CAM-Vorrichtung zum Abtragen von Material der Prothesenzähne mit einem CAM-Verfahren befestigt wird, wobei die Halteeinrichtung in der Halterung fixiert wird, wobei bevorzugt die Halteeinrichtung durch zwei Stifte gebildet wird, die an der Außenseite der Klemmvorrichtung angeordnet sind.

Die Halteeinrichtung dient der stabilen Verbindung zur CAM-Vorrichtung beziehungsweise einer einfachen und unaufwendigen Herstellbarkeit einer solchen stabilen Verbindung.

Ferner wird vorgeschlagen, dass die elastische Form einen Elastizitätsmodul von 10 MPa bis 5.000 MPa aufweist, bevorzugt einen Elastizitätsmodul von 50 MPa bis 2.000 MPa aufweist, besonders bevorzugt einen Elastizitätsmodul von 100 MPa bis 1.000 MPa aufweist.

Die elastische Form soll möglichst steif sein, um eine Veränderung der Position und der Lage der Prothesenzähne in der elastischen Form während des Abtragens von Material an der Prothesenbasis zu vermeiden. Insbesondere bei der Anwendung von Fräsmaschinen als CAM-Vorrichtungen bewirkt die Fräse eine mechanische Belastung der Prothesenzähne, durch die die Prothesenzähne nicht oder nicht zu sehr in ihrer Position und Ausrichtung zueinander verändert werden sollen. Dies ist sinnvoll, da ansonsten die Genauigkeit des CAM-Verfahrens reduziert wird, da die genaue Ausrichtung und Position Voraussetzung für CAM-Verfahren ist. Zu diesem Zweck sind die angegebenen Elastizitätsmodule besonders gut geeignet.

Des Weiteren kann erfindungsgemäß vorgesehen sein, dass die elastische Form aus einem Material besteht, das sich unter Druck verfestigt oder dass die elastische Form nach dem Einsetzen der Prothesenzähne verfestigt wird, bevorzugt durch Strahlung oder chemisch.

Hierdurch wird eine besonders stabile Fixierung der Prothesenzähne in der elastischen Form erreicht, wobei gleichzeitig ein leicht durchzuführendes Einsetzen der vorkonfektionierten Prothesenzähne in die Ausnehmungen der elastischen Form ermöglicht wird.

Es wird mit der Erfindung auch vorgeschlagen, dass eine CAM-gesteuerte Fräseinrichtung zum Durchführen des CAM-Verfahrens verwendet wird, bevorzugt eine CAM-gesteuerte Vier-Achs-Fräsmaschine.

Diese CAM-Vorrichtungen sind zur Umsetzung des Verfahrens besonders gut geeignet.

Bevorzugte erfindungsgemäße Verfahren können vorsehen, dass zumindest die äußeren Formen der basalen Seiten aller verwendeten vorkonfektionierten Prothesenzähne als erster Datensatz vorliegen und ein zweiter Datensatz die genaue Positionen und Ausrichtung aller in der elastischen Form fixierten Prothesenzähne definiert, wobei eine anschließende Bearbeitung des zumindest einen Prothesenzahns auf Basis des ersten und des zweiten Datensatzes durchgeführt wird, bevorzugt eine anschließende Bearbeitung mit einem CAD/CAM-Verfahren.

Hierdurch werden die Vorteile der erfindungsgemäßen Fixierung für vollautomatisierte Nachbearbeitungsverfahren zugänglich gemacht, für die sie besonders gut geeignet sind. Erst durch die beanspruchte Fixierung wird nämlich eine einfache und kostengünstige Bereitstellung der Prothesenzähne in einer hohen Genauigkeit und Positionstreue möglich.

Es wird auch vorgeschlagen, dass die verwendeten vorkonfektionierten Prothesenzähne vor dem Einsetzen in die elastische Form mit einem CAD-Verfahren hergestellt werden, wobei die Daten, die zur Bestimmung der äußeren Form der Oberfläche der vorkonfektionierten Prothesenzähne verwendet werden, zur Ausformung der Ausnehmungen in der elastischen Form verwendet werden, wobei vorzugsweise zumindest die Ausnehmungen in der elastischen Form mit einem CAD/CAM-Verfahren hergestellt werden.

Hierdurch wird eine weitere Automatisierung des erfindungsgemäßen Verfahrens erreicht.

Gemäß einer Weiterbildung erfindungsgemäßer Verfahren kann auch vorgesehen sein, dass vor dem Einsetzen der vorkonfektionierten Prothesenzähne in die Ausnehmungen der elastischen Form ein Haftvermittler in der Oberfläche der Ausnehmungen und/oder auf die Oberflächen der Prothesenzähne aufgetragen wird.

Hierdurch kann ein noch besserer Halt der Prothesenzähne in den Ausnehmungen der elastischen Form erreicht werden. Der Haftvermittler muss jedoch im Normalfall wieder von den Prothesenzähnen entfernt werden, wenn er nicht von selbst verdampft.

Gemäß einer bevorzugten Ausführung des erfindungsgemäßen Verfahrens kann auch vorgesehen sein, dass in Schritt 1) die Prothesenzähne derart in die Ausnehmungen der elastischen Form eingesetzt werden, dass die Ausnehmungen die Prothesenzähne an jeweils einem mittleren Umfang ringförmig vollständig umschließen.

Durch diese allseitige Fixierung der Prothesenzähne ist eine besonders stabile Lagerung, Ausrichtung und Positionierung der Prothesenzähne relativ zueinander möglich.

Die Aufgaben der Erfindung werden auch gelöst durch eine Vorrichtung zur Halterung von Prothesenzähnen zum Durchführen eines erfindungsgemäßen Verfahrens, wobei die Vorrichtung eine elastische Form mit wenigstens zwei Ausnehmungen zum Einsetzen von Prothesenzähnen aufweist und eine an der elastischen Form lösbar befestigbare Klemmvorrichtung zum Ausüben eines mechanischen Drucks auf die elastische Form aufweist, wobei die elastische Form zumindest eine Halteeinrichtung zur Verbindung der zusammengebauten Vorrichtung an einer CAD-Vorrichtung zum Abtragen von Material der eingesetzten Prothesenzähne aufweist.

Dabei kann vorgesehen sein, dass die Halteeinrichtung wenigstens ein Rastmittel aufweist, das in ein Gegenrastmittel einer CAM-Vorrichtung greift, wobei bevorzugt die Halteeinrichtung zumindest einen Stift mit zumindest einer umlaufenden Nut als Rastmittel aufweist, der in eine Halterungsöffnung einer CAM-Vorrichtung einsteckbar ist.

Mit dem Rastmittel ist ein einfaches und sicheres Verbinden der Vorrichtung mit der CAM-Vorrichtung möglich.

Weitere alternative mögliche Ausgestaltungen der Verbindungsmittel sind Klickverbindungen, Nut und Feder oder Steckverbindungen.

Auch bei der Vorrichtung kann erfindungsgemäß vorgesehen sein, dass die elastische Form einen Elastizitätsmodul von 10 MPa bis 5.000 MPa aufweist, bevorzugt einen Elastizitätsmodul von 50 MPa bis 2.000 MPa aufweist, besonders bevorzugt einen Elastizitätsmodul von 100 MPa bis 1.000 MPa aufweist.

Dadurch ergeben sich die oben genannten Vorteile.

Mit einer Weiterbildung der vorliegenden Erfindung wird vorgeschlagen, dass die Prothesenzähne nur in einer vorgegebenen Position und Ausrichtung in der elastischen Form fixierbar sind.

Es kann auch vorgesehen sein, dass die elastische Form eine zylindrische elastische Form ist. Dabei kann bevorzugt vorgesehen sein, dass die Höhe der zylindrischen elastischen Form zwischen 2 mm und 15 mm liegt. Zusätzlich oder alternativ kann vorgesehen sein, dass der Querschnitt oder der Durchmesser der zylindrischen elastischen Form zwischen 10 mm und 50 mm liegt.

Eine zylindrische Geometrie ist für die elastische Form besonders gut geeignet, da über die Mantelfläche auf besonders einfache Art und Weise der Druck der Klemmvorrichtung gleichmäßig auf die zylindrische elastische Form übertragen werden kann. Die bevorzugten Abmessungen sind zur Bearbeitung eines Prothesenzahns oder mehrerer Prothesenzähne gut geeignet.

Die der Erfindung zugrundeliegenden Aufgaben werden auch gelöst durch ein Set zur Durchführung eines solchen Verfahrens aufweisend wenigstens zwei vorkonfektionierte Prothesenzähne und wenigstens eine solche Vorrichtung.

Schließlich kann vorgesehen sein, dass das Set zusätzlich einen ersten Datensatz zur äußeren Form aller vorkonfektionierten Prothesenzähne und einen zweiten Datensatz zur Position der fixierten vorkonfektionierten Prothesenzähne in den Ausnehmungen der elastischen Form enthält, bevorzugt auf einem Datenträger.

Hierdurch wird eine vollautomatisierte Nachbearbeitung der Prothesenzähne vereinfacht.

Der Erfindung liegt die überraschende Erkenntnis zugrunde, dass es durch die Bearbeitung vorkonfektionierter Prothesenzähne, die in der bei der Prothese gewünschten Position und Ausrichtung zueinander fixiert werden, gelingt, die Prothesenzähne passend zueinander zu bearbeiten und dadurch passend zu der Prothesenbasis zu bearbeiten, so dass die erzeugte Prothese sehr exakt die gewünschten Abmessungen erhält. Gleichzeitig kann die Okklusion der Prothese mit noch vorhandenen Zähnen im Mundraum des Patienten oder die Okklusion zweier einander gegenüberliegenden Prothesen mit hoher Genauigkeit berücksichtigt und eingestellt werden. Mit der vorliegenden Erfindung können falsch aufgestellte Zähne und bezüglich der Okklusion schlecht sitzende Prothesen vermieden werden. Der Aufwand zum Einpassen erfindungsgemäß hergestellter Prothesen durch den Arzt wird verringert. Durch die Verfestigung des Halts der Prothesenzähne in den Ausnehmungen der elastischen Form wird es möglich, die Prothesenzähne in die zunächst noch nicht verspannte elastische Form einzusetzen und dabei die leichtere Verformbarkeit der elastischen Form dazu zu nutzen die Prothesenzähne in gut passende Ausnehmungen einzusetzen. Nach dem Ausüben des Drucks durch die Klemmvorrichtung nimmt die Verformbarkeit der elastischen Form ab und die Prothesenzähne werden stärker und damit für die Bearbeitung durch das CAM-Verfahren stabiler und Positions- und Ausrichtungsfester gehalten, so dass die Genauigkeit bei der automatisierten Bearbeitung der Prothesenzähne durch das CAM-Verfahren hoch ist.

Das Verfahren und die Vorrichtung und das Set sind besonders gut zur Bearbeitung mit CAD/CAM-Verfahren geeignet und zur Anwendung solcher Techniken vorgesehen. Die Befestigung der Prothesenzähne ist schnell und unkompliziert durchführbar. Die Klemmvorrichtung ist problemlos wiederverwendbar. Eine aufwendige Herstellung der Halterung, wie bei Wachshalterungen notwendig, entfällt bei dem erfindungsgemäßen Verfahren. Für den einen Patienten kann auch die elastische Form problemlos wiederverwendet werden, falls beispielsweise eine Reparatur der Prothese notwendig wird.

Die Klemmvorrichtung und die gesamte erfindungsgemäße Vorrichtung sind gegenüber äußeren Einflüssen wie Hitze oder Kälte stabil und es kommt nicht zum Verziehen oder zu Verformungen der Vorrichtung beziehungsweise der Klemmvorrichtung und damit nicht zu Positionsänderungen der Prothesenzähne innerhalb der Vorrichtung beziehungsweise innerhalb der gespannten beziehungsweise zusammengepressten elastischen Form.

Ein weiterer Vorteil erfindungsgemäßer Verfahren liegt in der sehr schnellen und nahezu werkzeug- und hilfsmittellosen Umsetzung. Dadurch kann auf das Vorhalten größerer Lagermengen verzichtet werden und eine bestellbezogene Konfektionierung vorgenommen werden. Auch entfällt die sonst bei Wachs notwendige Reinigung mittels Ausbrühen oder Putzen der Prothesenzähne.

Das Einbringen von Kontaktmitteln kann die Verbindung zwischen der elastischen Form und den Prothesenzähnen noch verbessern. Als beispielhaftes Kontaktmittel kann Polyvinylalkohol eingesetzt werden, das nach Abdampfen enthaltener Lösemittel eine haftende Verbindungsschicht bildet.

Die Erfindung kann beispielsweise vorsehen, dass die Prothesenzähne vom Anwender in die bereitgestellten Halter beziehungsweise der bereitgestellten elastischen Form der Vorrichtung so positioniert werden, dass sie im CAM-Verfahren maschinell geschliffen werden können und somit der individuellen Situation der Basisplatte beziehungsweise der Prothesenbasis und damit dem Patienten angepasst werden können. Primär wird dabei die basale Fläche der Prothesenzähne geschliffen, jedoch kann nach Befestigung der Prothesenzähne in der Prothesenbasis auch die okklusale Fläche zur individuellen Anpassung der Kaufunktion ebenfalls maschinell bearbeitet werden.

Hierzu müssen die konfektionierten Zähne in einer vordefinierten Position innerhalb eines in die Maschine einspannbaren Halters positioniert und festgehalten werden. Die Halter (die erfindungsgemäßen Vorrichtungen) sind dazu an die Anwendung solcher CAM-Verfahren angepasst, so dass sie einerseits die einzelnen Prothesenzähne ausreichend fest halten und dass sie andererseits stabil genug sind, um ohne Beschädigung aufeinander folgende Fräsprozesse überstehen zu können.

Die vorgefertigten konfektionierten Prothesenzähne werden dabei durch den Druck der Klemmvorrichtung von der elastischen Form in Presspassung gehalten.

Die elastische Form besteht bevorzugt aus weitgehend deformationsstabilem Material, zum Beispiel aus einem elastischen Kunststoffmaterial wie Silikon, Polymethylmethacrylat - PMMA oder Polyurethan - PU.

Die elastische Form weist erfindungsgemäß bevorzugt eine Dicke zwischen 2 mm und 15 mm auf. Bevorzugt wird hat die elastische Form die Geometrie eines allgemeinen Zylinders, der mit der Klemmvorrichtung an seiner Mantelfläche gedrückt wird.

Das erfindungsgemäße Verfahren bietet maximale Flexibilität bei der digitalen Herstellung von Teil- und Totalprothesen hinsichtlich der Prothesenzahnaufstellung. Mit dem Verfahren können klassische Konfektionszähne beziehungsweise klassische vorkonfektionierte Prothesenzähne verwendet werden, die in einem CAM-Verfahren von unterschiedlichen Maschinen in der elastischen Form bearbeitet werden können. Die beschliffenen Prothesenzähne können auch einzeln aus der elastischen Form entnommen werden.

Dadurch, dass bestehende konfektionierte Prothesenzähne verwendet werden können, können Prothesenzähne verwendet werden, die bereits bewährt sind und im Hinblick auf Funktion und Ästhetik im Markt anerkannt und beliebt sind. Das heißt, dass im Falle späterer Reparaturen einer Prothese klassische Konfektionsprothesenzähne gleicher Bauart auf Zahnleisten verwendet werden können. Es muss kein Block oder Rohling maschinell beschliffen werden, da der Zahntechniker die Prothesenzähne auch einzeln von der Zahnleiste nehmen und einsetzen kann.

Im Folgenden werden Ausführungsbeispiele der Erfindung anhand von drei schematisch dargestellten Figuren erläutert, ohne jedoch dabei die Erfindung zu beschränken. Dabei zeigt:
- Figur 1:: eine schematische Aufsicht auf eine erfindungsgemäße Vorrichtung ohne eingesetzte Prothesenzähne;
- Figur 2:: eine perspektivische Ansicht einer alternativen elastischen Form mit eingesetzten Prothesenzähnen zur Umsetzung eines erfindungsgemäßen Verfahrens;
- Figur 3:: eine schematische Aufsicht auf eine alternative erfindungsgemäße Vorrichtung, in der drei Prothesenzähne fixiert sind.

In den Figuren werden auch bei unterschiedlichen Ausführungen für gleichartige Teile teilweise die gleichen Bezugszeichen verwendet.

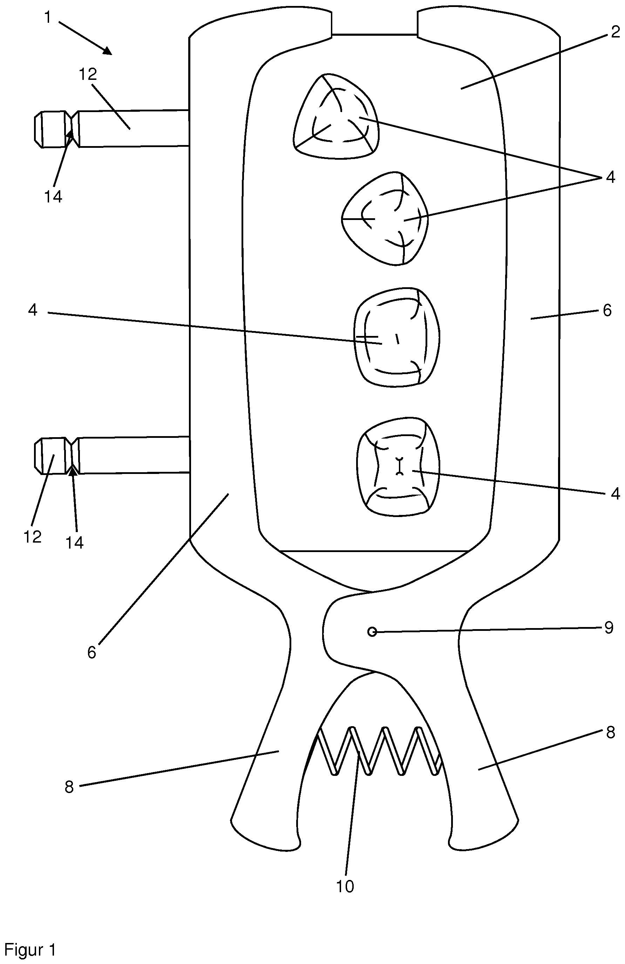

Figur 1 zeigt eine schematische Aufsicht auf eine erfindungsgemäße Vorrichtung 1, in der keine Prothesenzähne eingesetzt sind. Die gezeigte Prothesenzahnhalterung 1 hat eine elastische Form 2 aus einem Kunststoff, in dem vier Ausnehmungen 4 zur Aufnahme der koronalen Seiten von vorkonfektionierten Prothesenzähnen (nicht gezeigt) enthalten sind. Die Ausnehmungen 4 sind nicht durchgehend. Die elastische Form 2 ist bis auf die Ausnehmungen 4 als zylindrischer Block mit abgerundeter Grundfläche aufgebaut. In der Aufsicht nach Figur 1 fällt der Blick auf eine der ebenen Grundflächen des zylindrischen Blocks.

Die elastische Form 2 wird von einer Klemmvorrichtung 6 mit zwei Klemmschenkeln bereichsweise und weitgehend umfasst. Die beiden Klemmschenkel münden in einen Griff 8, so dass die Klemmvorrichtung 6 nach Art einer Wäscheklammer bedienbar ist. Die beiden Teile der Klemmvorrichtung 6 sind dazu über eine Achse 9 um diese drehbar mit einander verbunden. Eine elastische Stahlfeder 10 ist im Bereich des Griffs 8 angeordnet. Die Stahlfeder 10 drückt die beiden Teile des Griffs 8 auseinander und damit die Klemmschenkel zusammen beziehungsweise aufeinander zu. Dadurch wird auf den Zylindermantel der elastischen Form 2 ein Druck ausgeübt, der sich aufgrund der Elastizität des Materials bis zu den Ausnehmungen 4 fortsetzt. Dadurch können in die Ausnehmungen 4 eingesetzte Prothesenzähne (nicht gezeigt) fixiert werden. Die Ausrichtung und die Position der eingesetzten Prothesenzähne wird durch die Position und die Ausrichtung der Ausnehmungen 4 in der elastischen Form 2 bestimmt. Durch die Kraft, die von der Klemmvorrichtung 6 auf die elastische Form 2 ausgeübt wird, kann sich die elastische Form 2 verformen und dadurch die Position und Ausrichtung der fixierten Prothesenzähne von der der nicht fixierten Prothesenzähne unterscheiden.

Auf einer Seite der Klemmvorrichtung 6 sind zwei Stifte 12 als Halteeinrichtung befestigt, mit denen die gesamte Vorrichtung 1 in eine CAM-Fräsmaschine (nicht gezeigt) eingespannt beziehungsweise in einer CAM-Fräsmaschine befestigt werden kann. An den Stiften 12 sind umlaufende Nuten 14 als Rastmittel 14 vorgesehen, die in ein Gegenrastmittel (nicht gezeigt) der CAM-Fräsmaschine greifen und dadurch die gesamte Vorrichtung 1 in der CAM-Fräsmaschine ortsfest und positionsstabil fixieren.

Die vorkonfektionierten Prothesenzähne werden mit ihren koronalen Seiten beziehungsweise ihren koronalen Enden voraus in die Ausnehmungen 4 der elastischen Form 2 eingesteckt. Aufgrund der charakteristischen Form der Prothesenzähne können diese nur in einer Ausrichtung und Position eingesetzt werden. Beim Einsetzen können sich die Ausnehmungen 4 noch leichter verformen, so dass ein fester Sitz der Prothesenzähne in den Ausnehmungen 4 dadurch erreicht werden kann, dass sich bei der Rückverformung der Ausnehmungen 4 die Oberfläche der Ausnehmungen 4 formschlüssig an die koronalen Seiten der vollständig in die Ausnehmungen 4 eingesetzten Prothesenzähne anlegt. Anschließend wird die Klemmvorrichtung 6 angelegt und ein Druck auf die elastische Form 2 ausgeübt und dadurch die Prothesenzähne in der elastischen Form 2 fixiert.

Die gesamte Vorrichtung 1 wird mit Hilfe der Stifte 12 in die vollautomatische CAM-4-Achs-Fräsmaschine eingespannt. Anschließend werden die aus den Ausnehmungen 4 herausragenden basalen Seiten der Prothesenzähne abgeschliffen, so dass ihre basale Form der Oberfläche von Ausnehmungen einer Prothesenbasis entspricht, in die die Prothesenzähne eingesetzt werden sollen.

Durch die bekannten Abmessungen und die ortsfeste und positionsfeste Fixierung der Vorrichtung 1 und damit der darin fixierten vorkonfektionierten Prothesenzähne ist es möglich, dass die CAM-Fräsmaschine die basal aus den Ausnehmungen 4 und damit aus der Vorrichtung 1 herausragenden Prothesenzähne basierend auf einem virtuellen Computer-Modell (bevorzugt ein berechnetes CAD-Modell) basal abgefräst und an Ausnehmungen für diese Prothesenzähne in der Prothesenbasis angepasst werden, deren äußere Form ebenfalls bekannt ist und die zur Berechnung der durch Abfräsen zu erzielenden basalen Oberfläche der Prothesenzähne rechnerisch berücksichtigt wird.

Nachdem die Prothesenzähne bis zur gewünschten Form abgetragen wurden, können Sie noch in der Vorrichtung 1 gehaltert oder auch einzeln in der Prothesenbasis befestigt werden, indem die Prothesenzähne in die Prothesenbasis eingeklebt werden. Die korrekte Lage und Anordnung (beziehungsweise Position und Ausrichtung) wird bereits durch die Vorrichtung 1 gewährleistet. Anschließend wird die Klemmvorrichtung 6 entfernt und die elastische Form 2 von den Prothesenzähnen abgenommen.

Um die gewünschte Position und Ausrichtung der Prothesenzähne in der elastischen Form 2 zu erreichen, wird die elastische Form bevorzugt mit einem vollautomatischen CAD-CAM-Verfahren erzeugt und zwar basierend auf den gleichen Daten, die auch zur Herstellung und Bearbeitung der Prothesenbasis und der Prothesenzähne verwendet werden.

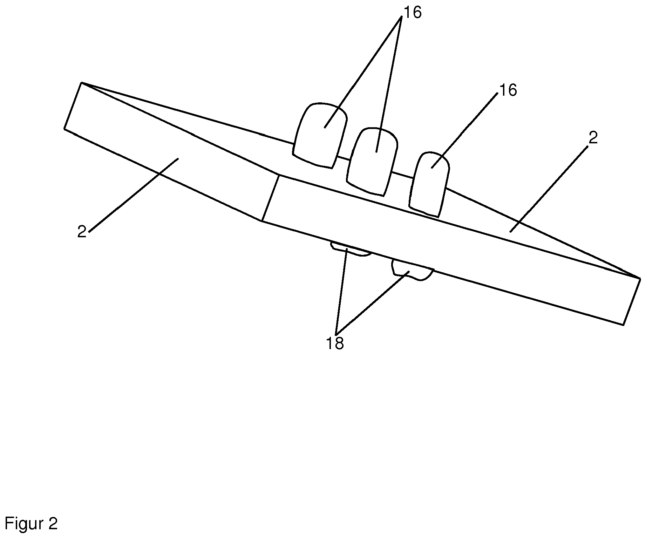

Figur 2 zeigt eine perspektivische Ansicht einer alternativen elastischen Form 2 mit eingesetzten Prothesenzähnen 16, 18 zur Umsetzung eines erfindungsgemäßen Verfahrens. Die in Figur 2 gezeigte elastische Form 2 unterscheidet sich von der nach Figur 1 dadurch, dass die Ausnehmungen in der elastischen Form 2 durchgehend sind, so dass bei den eingesetzten Prothesenzähnen 16, 18 sowohl die basalen Seiten 16 als auch die koronalen Seiten 18 zu sehen sind. Da hierdurch aber die Lagerung der Prothesenzähne 16, 18 verschlechtert wird, wird für eine solche elastische Form ein festerer Kunststoff beziehungsweise ein Kunststoff mit einem höheren Elastizitätsmodul bevorzugt. Zudem werden in dieser elastischen Form 2 nur drei Prothesenzähne 16, 18 eingesetzt. Die Elastische Form nach Figur 2 hat also nur drei Ausnehmungen, in denen die Prothesenzähne 16, 18 stecken.

Dadurch ist es möglich, sowohl die koronalen Seiten 18 als auch die basalen Seiten 16 der Prothesenzähne 16, 18 zu bearbeiten. Die Prothesenzähne 16, 18 werden bei der Ausführung nach Figur 2 also durch die Ausnehmungen der elastischen Form 2 gesteckt. Die Fixierung der Prothesenzähne 16, 18 erfolgt ebenso wie die nachfolgende Bearbeitung, wie zu Figur 1 geschildert, außer, dass eben beide Seiten 16, 18 bearbeitet werden können.

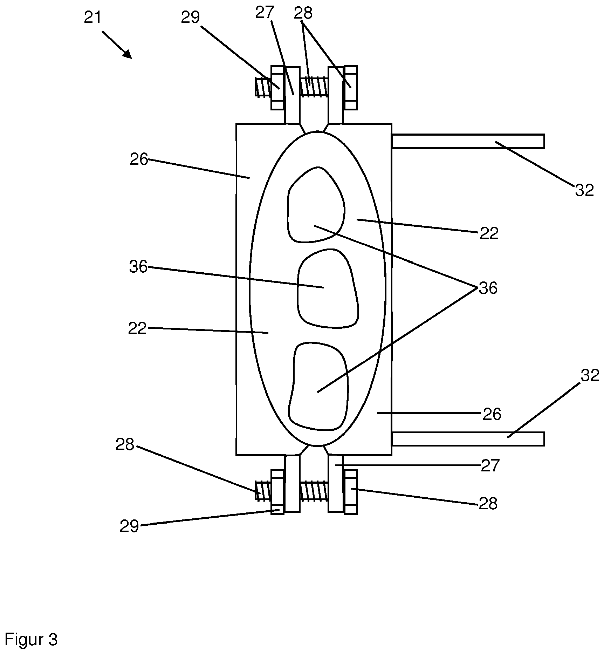

Figur 3 zeigt eine schematische Aufsicht auf eine alternative erfindungsgemäße Vorrichtung 21, in der drei Prothesenzähne 36 fixiert sind.

In der gezeigten Vorrichtung 21 sind drei Prothesenzähne 36 in drei Ausnehmungen (nicht zu sehen, da durch die Prothesenzähne 36 verdeckt) einer elastischen Form 22 fixiert. Die elastische Form 22 besteht aus einem Kunststoff und hat eine zylindrische Form mit ovalen Grundflächen. Durch die Aufsicht sind in Figur 3 die basalen Flächen der Prothesenzähne 36 also die basalen Seiten 36 der Prothesenzähne 36 zu erkennen. Die elastische Form 22 wird durch zwei Klemmbacken 26 einer Klemmvorrichtung 26 zusammengepresst. Im Unterschied zu der in Figur 1 gezeigten Ausführungsform erfolgt das Ausüben des Drucks auf die elastische Form nicht durch eine elastische Druckfeder sondern mit Hilfe eines Schraubensystems.

An jeweils zwei gegenüberliegenden Seiten der Klemmbacken 26 ist jeweils eine Lochplatte 27 befestigt, über die die Klemmbacken 26 aneinander befestigt werden können. Die Befestigung erfolgt mit je einer Schraube 28, die jeweils durch Löcher der Lochplatten 27 beider Klemmbacken 26 durchgesteckt ist und auf der Gegenseite durch je eine Kontermutter 29 fixiert wird.

Die Prothesenzähne 36 liegen formschlüssig und flächenbündig in je drei Ausnehmungen der elastischen Form 22 an. Durch die Beabstandung der beiden Klemmbacken 26 lastet der gesamte Druck auf der elastischen Form 22, die den Druck auf die Prothesenzähne 36 vermittelt, so dass eine besonders stabile Halterung erreicht wird. Der Druck kommt dabei vor allem aus zwei Richtungen (in Figur 3 von rechts und links), während die Haltekraft in der Richtung senkrecht dazu (in Figur 3 von oben und unten) schwächer als bei einer vollständig umgebenden Passform ist. Durch Einstellen des Drehmoments, mit dem die Schrauben 28 gegen die Kontermuttern 29 angezogen werden, kann eine definierte Kraft auf die elastische Form 22 und damit auf die Prothesenzähne 36 ausgeübt werden.

Wenn eine besonders stabile allseitige Fixierung mit noch gleichmäßigerer Kraftbeaufschlagung der Prothesenzähne 36 von allen Richtungen erreicht werden soll, kann auch eine Schelle nach Art einer Schlauchschelle zur Fixierung verwendet werden. Die Schelle umschließt dazu die elastische Form 22.

An der Klemmvorrichtung 26 sind zwei Stifte 32 angeordnet, mit denen die Vorrichtung 21 in einer CAM-Vorrichtung befestigt werden kann.

Es ist für die vorliegende Erfindung besonders vorteilhaft, wenn ein flächiger beziehungsweise formschlüssiger Druck auf die Prothesenzähnen 16, 18, 36 und die elastische Form 2, 22 auszuüben ist.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein. Es versteht sich, dass das Verfahren und die Vorrichtung für die gezeigten Anzahlen von Prothesenzähnen 16, 18, 36 neben den gezeigten Ausführungsbeispielen mit drei oder vier Prothesenzähnen 16, 18, 36 ohne weiteres auf andere Anzahlen von Prothesenzähnen 16, 18, 36 übertragbar sind. Bevorzugt können auch mehrere der gezeigten Prothesenzahnhalterungen 1, 21 beziehungsweise erfindungsgemäßen Vorrichtungen 1, 21 zur Umsetzung eines erfindungsgemäßen Verfahrens verwendet werden. Dann ist es möglich, die Okklusion von im Mundraum des Patienten eingesetzten gegenüberliegend angeordneten Prothesenzähnen 16, 18, 36 zu prüfen und einzustellen.

### Bezugszeichenliste

- 1, 21: Prothesenzahnhalterung
- 2, 22: Elastische Form
- 4: Ausnehmung mit koronaler Zahnform
- 6, 26: Klemmvorrichtung / Spange
- 8: Griff
- 9: Achse
- 10: Feder
- 12, 32: Stift / Halterung
- 14: Nut / Rastung
- 16, 36: Prothesenzahn basale Seite
- 18: Prothesenzahn koronale Seite
- 26: Klemmbacke
- 27: Lochplatte
- 28: Schraube
- 29: Mutter

## Patentansprüche

1. Verfahren zur Bearbeitung von Prothesenzähnen (16, 18, 36) zur Herstellung einer Totalprothese oder wenigstens einer Teilprothese mit zumindest zwei Prothesenzähnen (16, 18, 36) aufweisend die folgenden Verfahrensschritte:
1) Wenigstens zwei vorkonfektionierte Prothesenzähne (16, 18, 36) werden in eine einteilige elastische Form (2, 22) eingesetzt, wobei die koronalen Seiten (18) der Prothesenzähne (16, 18, 36) in Ausnehmungen (4) der elastischen Form (2, 22) eingesteckt werden,
2) eine Klemmvorrichtung (6, 26) wird an der elastischen Form (2, 22) angebracht oder ist an der elastischen Form (2, 22) angebracht und
mit der Klemmvorrichtung (6, 26) wird nach dem Einsetzen der Prothesenzähne (16, 18, 36) in die elastische Form (2, 22) ein mechanischer Druck auf die elastische Form (2, 22) ausgeübt, wobei der Druck über die elastische Form (2, 22) eine Kraft auf die in die elastische Form (2, 22) eingesetzten Prothesenzähne (16, 18, 36) ausübt und dadurch die Prothesenzähne (16, 18, 36) in der elastischen Form (2, 22) fixiert,
3) die Prothesenzähne (16, 18, 36) werden in der elastischen Form (2, 22) derart zueinander positioniert und ausgerichtet, dass, spätestens nach Anbringen der Klemmvorrichtung (6, 26) an der elastischen Form (2, 22), die Prothesenzähne (16, 18, 36) relativ zueinander die gewünschte Position und die gewünschte Ausrichtung aufweisen, die sie in der herzustellenden Teilprothese oder in der herzustellenden Totalprothese haben sollen,
4) die elastische Form (2, 22) wird mit der Klemmvorrichtung (6, 26) und den Prothesenzähnen (16, 18, 36) in einer definierten Position in einer Halterung einer CAM-Vorrichtung zum Abtragen von Material der Prothesenzähne (16, 18, 36) mit einem CAM-Verfahren befestigt, und
5) wenigstens einer der in der elastischen Form (2, 22) fixierten Prothesenzähne (16, 18, 36) wird mit einem CAM-Verfahren basal abgetragen.

2. Verfahren nach Anspruch 1, **dadurch gekennzeichnet, dass** die in der elastischen Form (2, 22) fixierten Prothesenzähne (16, 18, 36), nachdem zumindest einer der Prothesenzähne (16, 18, 36) basal abgetragen wurde, auf ihrer basalen Seite (16, 36) mit einer Prothesenbasis verbunden werden, wobei die Prothesenzähne (16, 18, 36) mit ihrer basalen Seite (16, 36) in dafür vorgesehene Ausnehmungen der Prothesenbasis geklebt werden.

3. Verfahren nach Anspruch 2, **dadurch gekennzeichnet, dass** nach dem Verbinden der Prothesenzähne (16, 18, 36) mit der Prothesenbasis die elastische Form (2, 22) von den Prothesenzähnen (16, 18, 36) gelöst wird, bevorzugt nach dem Verbinden der Prothesenzähne (16, 18, 36) mit der Prothesenbasis die Klemmvorrichtung (6, 26) gelöst wird oder von der elastischen Form (2, 22) gelöst wird und daran anschließend die elastische Form (2, 22) von den mit der Prothesenbasis verbundenen Prothesenzähnen (16, 18, 36) gelöst wird.

4. Verfahren nach einem der vorangehenden Ansprüche, **dadurch gekennzeichnet, dass**
die Prothesenzähne (16, 18, 36) beim Einsetzen in die elastische Form (2, 22) darin derart zueinander positioniert und ausgerichtet werden, dass die Prothesenzähne (16, 18, 36) zueinander die gewünschte Position und die gewünschte Ausrichtung aufweisen, die sie in der herzustellenden Teilprothese oder Totalprothese haben sollen.

5. Verfahren nach einem der vorangehenden Ansprüche, **dadurch gekennzeichnet, dass**
beim basalen Abtragen des zumindest einen Prothesenzahns (16, 18, 36) die durch das Abtragen erzeugte basale Oberfläche des zumindest einen Prothesenzahns (16, 18, 36) mit einem CAD-Verfahren berechnet wird.

6. Verfahren nach Anspruch 5, **dadurch gekennzeichnet, dass**
bei der Berechnung der zu erzeugenden basalen Oberfläche des zumindest einen Prothesenzahns (16, 18, 36) oder aller in der elastischen Form (2, 22) fixierten Prothesenzähne (16, 18, 36) die Oberfläche der Prothesenbasis, mit der die Prothesenzähne (16, 18, 36) verbunden werden sollen, und die bekannte Position und Ausrichtung des zumindest einen Prothesenzahns (16, 18, 36) relativ zu dem anderen Prothesenzahn (16, 18, 36) oder den anderen Prothesenzähnen (16, 18, 36) rechnerisch berücksichtigt wird.

7. Verfahren nach einem der Ansprüche 5 bis 6, **dadurch gekennzeichnet, dass** die gewünschte Okklusion der Prothesenzähne (16, 18, 36) zueinander oder der Prothesenzähne (16, 18, 36) und der echten Zähne des Patienten zueinander rechnergestützt bestimmt wird und bei einer Berechnung der basalen Oberfläche des CAD-Modells des zumindest einen Prothesenzahns (16, 18, 36) berücksichtigt wird.

8. Verfahren nach einem der vorangehenden Ansprüche, **dadurch gekennzeichnet, dass**
die Ausnehmungen (4) in der elastischen Form (2, 22) derart tief sind, dass sie die eingesetzten Prothesenzähne (16, 18, 36) an jeweils einem mittleren Umfang zumindest bereichsweise umschließen.

9. Verfahren nach einem der vorangehenden Ansprüche, **dadurch gekennzeichnet, dass**
die Innenflächen der Ausnehmungen (4) zumindest bereichsweise formschlüssig und flächenbündig zu Bereichen der Außenflächen der Prothesenzähne (16, 18, 36) passen, wobei die Innenflächen der Ausnehmungen (4) Negativen der Außenflächen der koronalen Seiten (18) der Prothesenzähne (16, 18, 36) entsprechen.

10. Verfahren nach einem der vorangehenden Ansprüche, **dadurch gekennzeichnet, dass**
die elastische Form (2, 22) einen Elastizitätsmodul von 10 MPa bis 5.000 MPa aufweist, bevorzugt einen Elastizitätsmodul von 50 MPa bis 2.000 MPa aufweist, besonders bevorzugt einen Elastizitätsmodul von 100 MPa bis 1.000 MPa aufweist.

11. Verfahren nach einem der vorangehenden Ansprüche, **dadurch gekennzeichnet, dass**
die elastische Form (2, 22) aus einem Material besteht, das sich unter Druck verfestigt oder dass die elastische Form (2, 22) nach dem Einsetzen der Prothesenzähne (16, 18, 36) verfestigt wird, bevorzugt durch Strahlung oder chemisch.

12. Verfahren nach einem der vorangehenden Ansprüche, **dadurch gekennzeichnet, dass**
zumindest die äußeren Formen der basalen Seiten (16, 36) aller verwendeten vorkonfektionierten Prothesenzähne (16, 18, 36) als erster Datensatz vorliegen und ein zweiter Datensatz die genaue Positionen und Ausrichtung aller in der elastischen Form (2, 22) fixierten Prothesenzähne (16, 18, 36) definiert, wobei eine anschließende Bearbeitung des zumindest einen Prothesenzahns (16, 18, 36) auf Basis des ersten und des zweiten Datensatzes durchgeführt wird, bevorzugt eine anschließende Bearbeitung mit einem CAD/CAM-Verfahren.

13. Verfahren nach einem der vorangehenden Ansprüche, **dadurch gekennzeichnet, dass**
in Schritt 1) die Prothesenzähne (16, 18, 36) derart in die Ausnehmungen (4) der elastischen Form (2, 22) eingesetzt werden, dass die Ausnehmungen (4) die Prothesenzähne (16, 18, 36) an jeweils einem mittleren Umfang ringförmig vollständig umschließen.

14. Vorrichtung (1, 21) zur Halterung von Prothesenzähnen (16, 18, 36) zum Durchführen eines Verfahrens nach einem der vorangehenden Ansprüche, **dadurch gekennzeichnet, dass**
die Vorrichtung (1, 21) eine einteilige elastische Form (2, 22) mit wenigstens zwei Ausnehmungen (4) zum Einsetzen von Prothesenzähnen (16, 18, 36) aufweist und eine an der elastischen Form (2, 22) lösbar befestigbare Klemmvorrichtung (6, 26) zum Ausüben eines mechanischen Drucks auf die elastische Form (2, 22) aufweist, wobei die elastische Form (2, 22) zumindest eine Halteeinrichtung (12, 32) zur Verbindung der zusammengebauten Vorrichtung an einer CAM-Vorrichtung zum Abtragen von Material der eingesetzten Prothesenzähne (16, 18, 36) aufweist.

15. Vorrichtung (1, 21) nach Anspruch 14, **dadurch gekennzeichnet, dass** die Halteeinrichtung (12, 32) wenigstens ein Rastmittel (14) aufweist, das in ein Gegenrastmittel einer CAM-Vorrichtung greift, wobei die Halteeinrichtung (12, 32) zumindest einen Stift (12, 32) mit zumindest einer umlaufenden Nut (14) als Rastmittel (14) aufweist, der in eine Halterungsöffnung einer CAM-Vorrichtung einsteckbar ist.

16. Vorrichtung (1, 21) nach Anspruch 14 oder 15, **dadurch gekennzeichnet, dass** die Prothesenzähne (16, 18, 36) nur in einer vorgegebenen Position und Ausrichtung in der elastischen Form (2, 22) fixierbar sind.

17. Set zur Durchführung eines Verfahrens nach einem der Ansprüche 1 bis 13 aufweisend wenigstens zwei vorkonfektionierte Prothesenzähne (16, 18, 36), und wenigstens eine Vorrichtung (1, 21) nach einem der Ansprüche 14 bis 16.

18. Set nach Anspruch 17, **dadurch gekennzeichnet, dass**
das Set zusätzlich einen ersten Datensatz zur äußeren Form aller vorkonfektionierten Prothesenzähne (16, 18, 36) und einen zweiten Datensatz zur Position der fixierten vorkonfektionierten Prothesenzähne (16, 18, 36) in den Ausnehmungen (4) der elastischen Form (2, 22) enthält, bevorzugt auf einem Datenträger.

## Claims

1. A method for processing prosthetic teeth (16, 18, 36) for the production of a full denture or at least a partial denture with at least two prosthetic teeth (16, 18, 36) comprising the following method steps:
1) inserting at least two prefabricated prosthetic teeth (16, 18, 36) into an one-piece elastic mould (2, 22), wherein the coronal sides (18) of the prosthetic teeth (16, 18, 36) are inserted into recesses (4) in the elastic mould (2, 22),
2) attaching a clamping device (6, 26) to the elastic mould (2, 22) or the clamping device (6, 26) being already attached to the elastic mould (2, 22), and
after insertion of the prosthetic teeth (16, 18, 36) into the elastic mould (2, 22) applying a mechanical pressure to the elastic mould (2, 22) with the clamping device (6, 26), wherein the pressure exerts a force onto the prosthetic teeth (16, 18, 36) inserted into the elastic mould (2, 22) via the elastic mould (2, 22) and thereby affixing the prosthetic teeth (16, 18, 36) in the elastic mould (2, 22),
3) positioning and aligning the prosthetic teeth (16, 18, 36) in the elastic mould (2, 22) in such a manner in relation to each other that at the latest following application of the clamping device (6, 26) on the elastic mould (2, 22), the prosthetic teeth (16, 18, 36) comprise the desired position and desired alignment in relation to each other which they should have in the partial denture to be produced or full denture to be produced,
4) affixing the elastic mould (2, 22) with the clamping device (6, 26) and prosthetic teeth (16, 18, 36) in a defined position in a holder of a CAM device for removing material of the prosthetic teeth (16, 18, 36) with a CAM method, and
5) basally ablating at least one of the prosthetic teeth (16, 18, 36) affixed in the elastic mould (2, 22) with a CAM method.

2. The method according to claim 1, **characterized in that**
the prosthetic teeth (16, 18, 36) affixed in the elastic mould (2, 22) are connected to a denture base on their basal side (16, 36) after at least one of the prosthetic teeth (16, 18, 36) has been basally ablated, wherein the prosthetic teeth (16, 18, 36) are glued with their basal side (16, 36) in recesses in the denture base provided for the purpose.

3. The method according to claim 2, **characterized in that**
following the connection of the prosthetic teeth (16, 18, 36) with the denture base, the elastic mould (2, 22) is released from the prosthetic teeth (16, 18, 36), preferably after connecting the prosthetic teeth (16, 18, 36) with the denture base, the clamping device (6, 26) is released or released from the elastic mould (2, 22), and then subsequently, the elastic mould (2, 22) is released from the prosthetic teeth (16, 18, 36) which are connected to the denture base.

4. The method according to any one of the above claims, **characterized in that** the prosthetic teeth (16, 18, 36) are positioned and aligned during insertion into the elastic mould (2, 22) in such a manner that the prosthetic teeth (16, 18, 36) comprise the desired position and desired orientation in relation to each other which they should have in the partial denture or full denture to be produced.

5. The method according to any one of the above claims, **characterized in that** during basal ablation of at least one prosthetic tooth (16, 18, 36), the basal surface of the at least one prosthetic tooth (16, 18, 36) which is generated by the ablation is calculated using a CAD method.

6. The method according to claim 5, **characterized in that**
when calculating the basal surface to be created on the at least one prosthetic tooth (16, 18, 36) or on all the prosthetic teeth (16, 18, 36) affixed in the elastic mould (2, 22) the surface of the denture base with which the prosthetic teeth (16, 18, 36) are to be connected and the known position and alignment of the at least one prosthetic tooth (16, 18, 36) relative to the other prosthetic tooth (16, 18, 36) or the other prosthetic teeth (16, 18, 36) is taken into account in the calculation.

7. The method according to any one of claims 5 to 6, **characterized in that**
the desired occlusion of the prosthetic teeth (16, 18, 36) in relation to each other or the prosthetic teeth (16, 18, 36) and the real teeth of the patient in relation to each other is determined in a computer-supported manner and is taken into account when calculating the basal surface of the CAD model of the at least one prosthetic tooth (16, 18, 36).

8. The method according to any one of the preceding claims, **characterized in that** the recesses (4) in the elastic mould (2, 22) are sufficiently deep that they enclose the inserted prosthetic teeth (16, 18, 36) at least in sections at a central circumference respectively.

9. The method according to any one of the preceding claims, **characterized in that** the inner surfaces of the recesses (4) are at least in sections match in a form-fit and flush-mounted to areas of the outer surfaces of the prosthetic teeth (16, 18, 36), wherein the inner surfaces of the recesses (4) correspond to negatives of the outer surfaces of the coronal sides (18) of the prosthetic teeth (16, 18, 36).

10. The method according to any one of the preceding claims, **characterized in that** the elastic mould (2, 22) comprises an elasticity module of 10 MPa to 5,000 MPa, preferably an elasticity module of 50 MPa to 2,000 MPa, and in a particularly preferred manner, from 100 MPa to 1,000 MPa.

11. The method according to any one of the preceding claims, **characterized in that** the elastic mould (2, 22) consists of a material which rigidifies under pressure, or that the elastic mould (2, 22) is affixed following insertion of the prosthetic teeth (16, 18, 36), preferably through radiation or chemically.

12. The method according to any one of the preceding claims, **characterized in that** at least the outer forms of the basal sides (16, 36) of all prefabricated prosthetic teeth (16, 18, 36) used are present as a first data set, and a second data set defines the precise positions and alignment of all prosthetic teeth (16, 18, 36) affixed in the elastic mould (2, 22), wherein a subsequent processing of the at least one prosthetic tooth (16, 18, 36) is conducted on the basis of the first and second data set, preferably subsequent processing with a CAD/CAM method.

13. The method according to any one of the preceding claims, **characterized in that** in step 1), the prosthetic teeth (16, 18, 36) are inserted into the recesses (4) of the elastic mould (2, 22) in such a manner that the recesses (4) fully enclose the prosthetic teeth (16, 18, 36) on a central circumference respectively.

14. A device (1, 21) for holding prosthetic teeth (16, 18, 36) for implementing a method according to the any one of the preceding claims, **characterized in that** the device (1, 21) comprises an one-piece elastic mould (2,22) with at least two recesses (4) for inserting prosthetic teeth (16, 18, 36) and a clamping device (6, 26) which can be detachably affixed to the elastic mould (2, 22) for exerting a mechanical pressure onto the elastic mould (2, 22), wherein the elastic mould (2, 22) comprises at least one retaining installation (12, 32) for connecting the constructed device to a CAM device for removing material of the prosthetic teeth (16, 18, 36) inserted.

15. The device (1, 21) according to claim 14, **characterized in that** the retaining installation (12, 32) comprises at least one latching means (14), which grips into a counter latching means of a CAM device, wherein preferably, the retaining installation (12, 32) comprises at least one pin (12, 32) with at least one circumferential groove (14) as a latching means (14), which can be inserted into a holder opening of a CAM device.

16. The device (1, 21) according to claim 14 or 15, **characterized in that** the prosthetic teeth (16, 18, 36) can only be affixed in a specified position and alignment in the elastic mould (2, 22).

17. A set for implementing the method according to any one of claims 1 to 13, comprising at least two prefabricated prosthetic teeth (16, 18, 36) and at least one device (1, 21) according to any one of claims 14 to 16.

18. A set according to claim 17, **characterized in that**
the set additionally contains a first data set relating to the outer form of all prefabricated prosthetic teeth (16, 18, 36) and a second data set relating to the position of the affixed prefabricated prosthetic teeth (16, 18, 36) in the recesses (4) of the elastic mould (2, 22), preferably on a data storage device.

## Revendications

1. Procédé de façonnage de dents prothétiques (16, 18, 36) pour la fabrication d'une prothèse totale ou d'au moins une prothèse partielle avec au moins deux dents prothétiques (16, 18, 36), présentant les étapes de procédé suivantes :
1) au moins deux dents prothétiques (16,18, 36) pré-confectionnées sont implantées dans une forme élastique (2, 22) en une seule pièce, où les faces coronales (18) des dents prothétiques (16, 18, 36) sont implantées dans des cavités (4) de la forme élastique (2, 22),
2) un dispositif de serrage (6, 26) est rapporté sur la forme élastique (2, 22) ou est agencé sur la forme élastique (2, 22), et après l'implantation des dents prothétiques (16, 18, 36) dans la forme élastique (2, 22), une pression mécanique est exercée sur la forme élastique (2, 22) avec le dispositif de serrage (6, 26), où la pression exerce une force sur les dents prothétiques (16, 18, 26) implantées dans la forme élastique (2, 22), par le biais de la forme élastique (2, 22), et fixe ainsi les dents prothétiques (16, 18, 36) dans la forme élastique (2, 22),
3) les dents prothétiques (16, 18, 36) sont positionnées et orientées les unes par rapport aux autres dans la forme élastique (2, 22) de telle manière qu'au plus tard après la mise en place du dispositif de serrage (6, 26) sur la forme élastique (2, 22), les dents prothétiques (16, 18, 36) présentent la position souhaitée et l'orientation souhaitée les unes par rapport aux autres qu'elles doivent avoir dans la prothèse partielle à fabriquer ou dans la prothèse totale à fabriquer,
4) la forme élastique (2, 22) est fixée avec le dispositif de serrage (6, 26) et les dents prothétiques (16, 18, 36) dans une position définie dans un support d'un dispositif de FAO pour un retrait de matière des dents prothétiques (16, 18, 36) avec un procédé FAO, et
5) au moins une des dents prothétiques (16, 18, 36) fixées dans la forme élastique (2, 22) est soumise à un retrait à la base avec un procédé FAO.

2. Procédé selon la revendication 1, **caractérisé en ce que**
les dents prothétiques (16, 18, 36) fixées dans la forme élastique (2, 22) sont reliées sur leurs faces de base (16, 36) avec une base de prothèse après qu'au moins une des dents prothétiques (16, 18, 36) ait été soumise à un retrait à la base, où les dents prothétiques (16, 18, 36) sont collées avec leur face de base (16, 36) dans des cavités de la base de prothèse prévues à cet effet.

3. Procédé selon la revendication 2, **caractérisé en ce**
**qu'**après la liaison des dents prothétiques (16, 18, 36) avec la base de prothèse, la forme élastique (2, 22) est détachée des dents prothétiques (16, 18, 36), de préférence, après la liaison des dents prothétiques (16, 18, 36) avec la base de prothèse, le dispositif de serrage (6, 26) est détaché ou est détaché de la forme élastique (2, 22) et ensuite la forme élastique (2, 22) est détachée des dents prothétiques (16, 18, 36) reliées avec la base de prothèse.

4. Procédé selon l'une des revendications précédentes, **caractérisé en ce que**
les dents prothétiques (16, 18, 36) sont positionnées et orientées les unes par rapport aux autres de telle manière que les dents prothétiques (16, 18, 36) présentent la position souhaitée et l'orientation souhaitée les unes par rapport aux autres qu'elles doivent avoir dans la prothèse partielle ou dans la prothèse totale à fabriquer.

5. Procédé selon l'une des revendications précédentes, **caractérisé en ce que**
lors du retrait à la base de l'au moins une dent prothétique (16, 18, 36), la surface de base créée par le retrait de l'au moins une dent prothétique (16, 18, 36) est calculée avec un procédé FAO.

6. Procédé selon la revendication 5, **caractérisé en ce que**
lors du calcul de la surface à la base de l'au moins une dent prothétique (16, 18, 36) à créer ou de toutes les dents prothétiques (16, 18, 36) fixées dans la forme élastique (2, 22), la surface de la base de prothèse, avec laquelle les dents prothétiques (16, 18, 36) doivent être reliées et la position et l'orientation de l'au moins une dent prothétique (16, 18, 36) doivent être calculées par rapport à l'autre dent prothétique (16, 18, 36) ou aux autres dents prothétiques (16, 18, 36).

7. Procédé selon l'une des revendications 5 à 6, **caractérisé en ce que**
l'occlusion souhaitée des dents prothétiques (16, 18, 36) les unes par rapport aux autres ou des dents prothétiques (16, 18, 36) et des dents véritables du patient les unes par rapport aux autres, est déterminée en s'appuyant sur des calculs et est prise en compte dans le calcul de la surface de base du modèle de FAO de l'au moins une dent prothétique (16, 18, 36).

8. Procédé selon l'une des revendications précédentes, **caractérisé en ce que**
les cavités (4) dans la forme élastique (2, 22) sont à une profondeur telle qu'elles entourent au moins par endroits les dents prothétiques (16, 18, 36) implantées au niveau de leur périmètre moyen respectif.

9. Procédé selon l'une des revendications précédentes, **caractérisé en ce que**
les surfaces intérieures des cavités (4) s'ajustent au moins par endroits par complémentarité des formes et de manière à être en affleurement avec des zones des surfaces extérieures des dents prothétiques (16, 18, 36), où les surfaces intérieures des cavités (4) correspondent à des négatifs des surfaces extérieures des faces coronales (18) des dents prothétiques (16, 18, 36).

10. Procédé selon l'une des revendications précédentes, **caractérisé en ce que**
la forme élastique (2, 22) présente un module d'élasticité de 10 MPa à 5000 MPa, de préférence un module d'élasticité de 50 MPa à 2000 MPa, de manière particulièrement préférée, un module d'élasticité de 100 MPa à 1000 MPa.

11. Procédé selon l'une des revendications précédentes, **caractérisé en ce que**
la forme élastique (2, 22) est constituée d'un matériau qui se solidifie sous la pression ou que la forme élastique (2, 22) est solidifiée après l'implantation des dents prothétiques (16, 18, 36), de préférence par un rayonnement ou de manière chimique.

12. Procédé selon l'une des revendications précédentes, **caractérisé en ce**
**qu'**au moins les formes extérieures des faces de base (16, 36) de toutes les dents prothétiques (16, 18, 36) pré-confectionnées utilisées se présentent sous la forme d'un premier ensemble de données et un deuxième ensemble de données définit les positions exactes et l'orientation de toutes les dents prothétiques (16, 18, 36) fixées dans la forme élastique (2, 22), où le façonnage ultérieur de l'au moins une dent prothétique (16, 18, 36) est effectué sur la base des premier et deuxième ensembles de données, de préférence, un façonnage ultérieur avec un procédé CAO/FAO.

13. Procédé selon l'une des revendications précédentes, **caractérisé en ce que**
dans l'étape 1), les dents prothétiques (16, 18, 36) sont implantées dans les cavités (4) de la forme élastique (2, 22) de telle manière que les cavités (4) entourent totalement, comme une bague, les dents prothétiques (16, 18, 36) au niveau de leur périmètre moyen respectif.

14. Dispositif (1, 21) de maintien de dents prothétiques (16, 18, 36) pour l'exécution d'un procédé selon l'une des revendications précédentes, **caractérisé en ce que**
le dispositif (1, 21) présente une forme élastique (2, 22) en une pièce avec au moins deux cavités (4) pour l'implantation de dents prothétiques (16, 18, 36) et présente un dispositif de serrage (6, 26) pouvant être fixé de manière amovible sur la forme élastique (2, 22) pour l'exercice d'une pression mécanique sur la forme élastique (2, 22), où la forme élastique (2, 22) présente au moins un dispositif de maintien (12, 32) pour la liaison du dispositif monté sur un dispositif de FAO pour le retrait de matière des dents prothétiques (16, 18, 36) implantées.

15. Dispositif (1, 21) selon la revendication 14, **caractérisé en ce que**
le dispositif de maintien (12, 32) présente au moins un moyen de verrouillage (14) qui se met en prise avec un moyen de verrouillage complémentaire d'un dispositif FAO, où le dispositif de maintien (12, 32) présente au moins une tige (12, 32) avec au moins une rainure circonférentielle (14) servant de moyen de verrouillage (14), qui peut être inséré dans un orifice de maintien d'un dispositif FAO.

16. Dispositif (1, 21) selon la revendication 14 ou la revendication 15, **caractérisé en ce que**
les dents prothétiques (16, 18, 36) ne peuvent être fixées que dans une position et une orientation prédéfinies dans la forme élastique (2, 22).

17. Ensemble prévu pour l'exécution d'un procédé selon l'une des revendications 1 à 13 présentant au moins deux dents prothétiques (16, 18, 36) pré-confectionnées et au moins un dispositif (1, 21) selon l'une des revendications 14 à 16.

18. Ensemble selon la revendication 17, **caractérisé en ce que**
l'ensemble contient de manière complémentaire un premier ensemble de données pour la forme extérieure de toutes les dents prothétiques (16, 18, 36) et un deuxième ensemble de données pour la position des dents prothétiques (16, 18, 36) pré-confectionnées fixées dans les cavités (4) de la forme élastique (2, 22), de préférence sur un support de données.
